# EUROPEAN PATENT SPECIFICATION

(11) **EP 2 085 085 B1**
(45) Date of publication and mention of the grant of the patent: **17.10.2012**
(21) Application number: 07763981.3
(22) Date of filing: 04.07.2007
(51) Int. Cl.: A61K 31/4192, A61P 29/00, A61K 45/06

(54) **THE USES OF THE CARBOXY-AMIDO-TRIAZOLE COMPOUNDS AND SALTS THEREOF**
VERWENDUNGEN VON CARBOXY-AMIDO-TRIAZOL-VERBINDUNGEN UND IHREN SALZEN
UTILISATIONS DE COMPOSÉS CARBOXY-AMIDO-TRIAZOLES ET DE LEURS SELS

(30) Priority: 16.10.2006 CN 200610113770
(43) Date of publication of application: 05.08.2009
(73) Proprietor: Institute of Basic Medical Sciences Chinese Academy of Medical Sciences, Beijing 100-005 (CN)
(72) Inventor: ZHANG, Dechang, Beijing 100005 (CN); YE, Caiying, Beijing 100005 (CN); GUO, Lei, Beijing 100005 (CN); CHEN, Wenying, Beijing 100005 (CN)
(74) Representative: Ahner, Francis
(86) International application number: PCT/CN2007/002069
(87) International publication number: WO 2008/046285

(56) References cited:
- EP-A2- 0 304 221
- WO-A1-90/14009
- WO-A1-93/25536
- WO-A1-95/07695
- WO-A1-96/18394
- WO-A2-2006/037106
- FRANKLIN ALAN J ET AL: "CAI is a potent inhibitor of neovascularization and imparts neuroprotection in a mouse model of ischemic retinopathy" IOVS, vol. 45, no. 10, October 2004 (2004-10), pages 3756-3766, XP002577445
- WINTERS MARY E ET AL: "Supra-additive growth inhibition by a celecoxib analogue and carboxyamido-triazole is primarily mediated through apoptosis" CANCER RESEARCH, vol. 65, no. 9, 1 May 2005 (2005-05-01), pages 3853-3860, XP002577451 ISSN: 0008-5472

## Description

### FIELD OF THE INVENTION

The present invention relates to the new medical use of carboxyamidotriazole (CAI), and pharmaceutical salts thereof, analogues or derivatives and morphine in the preparation of the medicaments for treating the diseases mediated by cytokines TNF-α and IL-1β(inflammatory cytokines), in particular painful diseases and/or inflammatory diseases;especially the use for preparation the medicaments for treating inflammatory diseases involving inflammatory cytokines, chronic rheumatic diseases and other related inflammatory immune-mediated diseases.

### BACKGROUND OF THE INVENTION

Among a variety of diseases, cancer is one of the most common fatal diseases. Recent studies demonstrated that chronic inflammation is associated with the formation and development of cancer. Fundamentally, cancer is the final stage of many types of chronic inflammatory diseases. It provides continuous blood vessel support and substance support for ecphyma that is formed. It has been confirmed that tumor microenvironment is mainly regulated by inflammatory cells which are involved in tumor formation, cell proliferation, survival and migration. In addition, one skilled in the art is aware that patients in late stage of cancer usually suffer from severe pains. An anticancer drug with both antitumor and anti-inflammatory and analgesic effects would broaden its use in clinic and increase its therapeutic effects. In addition, such drug would also be helpful for discovering the role of inflammation in the formation and development of tumor and, it may be developed as novel anti-tumor drugs targeting inflammation, as well as novel anti-inflammatory drugs.

Carboxyamidotriazole, 5-amino-1-[3, 5-dichloro-4-(4-chlorobenzoyl chloride) benzyl]-1H-1, 2, 3-triazole-4-carboxamide is a new anti-tumor drug. Its structure is as follow:

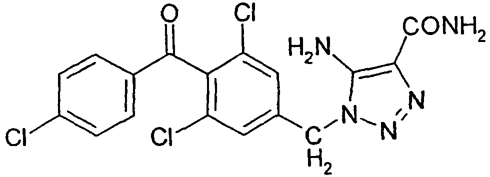

E.C.Kohn et al disclosed the anti-tumor activity of carboxyamidotriazole in European patent EP0644880(1995). It is believed that the mechanism of anti-tumor effect of CAI is to interfere calcium channel-mediated signal transduction of tumor cells and to inhibit tumor angiogenesis. Currently, there is no report about anti-inflammatory and analgesic effects of CAI.

In addition, according to the current opinions in immunopathology, TNF-α and IL-1β play key roles in many immune and inflammation diseases. Large amounts of evidence have demonstrated the important roles of TNF-α and IL-1β in pathologic processes of rheumatoid arthritis (RA). TNF-α exists in a variety of cells of RA synovial membrane, especially in the junction of blood vessels and cartilage. TNF-α is involved in inflammatory reaction of synovial membrane and induces the ruin of joint structure. IL-1β activates osteoclast, suppresses the synthesis of joint collagen and glycoprotein, enables absorption of the cartilage and bone, and causes the joint loss its integrity and functions. TNF-α is also a major mediator of septic shock. TNF-α and other cytokines trigger inflammatory and metabolic response associated with sepsis and septic shock, such as adult distress of respiratory (ADRS), fever and disseminated intravascular coagulation. The cytokines mentioned above are also involved in the fever, culminating emaciation and mycobacterium tuberculosis infection of AIDS patients. TNF-α and IL-1β are also involved in many other diseases/symptoms, such as injury of blood vessel/ atherosclerosis, type I diabetes, , Kawasaki disease, , leprosy, , disseminated sclerosis, chronic anemia, , ultraviolet radiation, , HP gastritis / canker, , Brazilian blastomycosis, septic melioidosis, , cardiac failure, , familial Mediterranean fever, toxic shock, chronic fatigue, homograft rejection, , Graft-versus-host disease, , schistosomiasis and so on.

Therefore, based on the above knowledge about pro-inflammation mediators(TNF-α and IL-1β), their inhibitors are possible to be used as new drugs for prevention and treatment of painful diseases, inflammatory diseases, especially for the treatment of cytokine-mediated inflammation, chronic rheumatic disease and other immune diseases.

Through a long term and intensive investigation, the inventors find that carboxyamidotriazole, which has long been recognized as an anti-cancer drug, is a potent inhibitor of pro-inflammatory cytokines (TNF-α and IL-1β) and that it has analgesic and anti-inflammatory effect.

### DETAILED DESCRIPTION OF THE INVENTION

The instant invention provides use of carboxyamidotriazole and pharmaceutical salts thereof, analogues or derivatives as TNF-α and IL-linhibitor and morphine in preparation of a medicament for treating painful diseases and/or inflammatory diseases.

In the present invention, carboxyamidotriazole compounds are :
(1) compounds of Formula I : 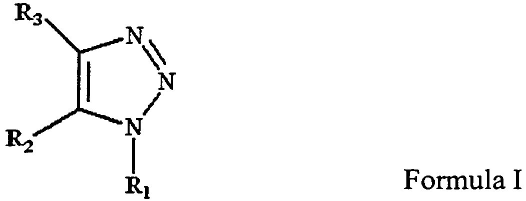 wherein R1 has a structure of Formula II : 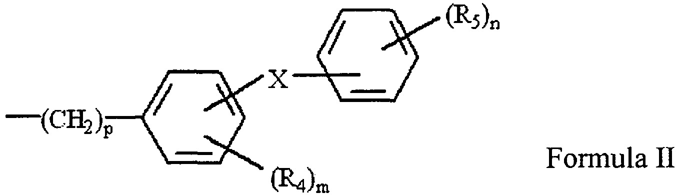 wherein, p is an integer from 0 to 2 ;m is an integer from 0 to 4 ;n is an integer from 0 to 5 ;X may be O, S, SO, SO₂, CO, CHCN, CH₂ or C = NR₆
   wherein R₆ may be H, (C₁-C₆) alkyl, hydroxy, (C₁-C₆) alkoxyl, amino, (C₁-C₆) alkylamino, dialkylamino or cyano ;
   R₄ and R₅ are independently halogen, cyano, trifluoromethyl, (C₁-C₆) alkyl acyl, nitro, (C₁-C₆)alkyl, (C₁-C₆) alkoxyl, carbonyl, alkyl ester, trifluoromethoxyl, acetyl amino, (C₁-C₆) alkyl thiol, (C₁-C₆) alkyl sulfuryl, trichloroethylene, trifluoromethylthio, trifluoromethylsulfinyl, trifluoromethylsulfuryl ;
   R₂ is amido, (C₁-C₆)alkylamino, dialkylamino, acetyl amino, acetylimino, acyl ureido, formamido, formylimino or guanidino ;
   R3 is amino formyl, cyano, formamyl, imino or N-hydroxy formamyl.
(2)particularly, in Formula II, n and m are independently 0, 1 or 2 ; P is 1 ; X is O, S, CO or CH₂ ;
   R₄ is F, Cl, Br, methyl, trifluoromethyl, cyano, methoxycarbonyl, trifluoromethoxy, trifluoromethylthio, nitro or trichloroethylene ;
   R₅ is F, Cl, Br, methyl, trifluoromethyl, cyano, alkyl ester, trifluoroethylene or nitro.
(3) More particularly, compounds of Formula I has the following structure: 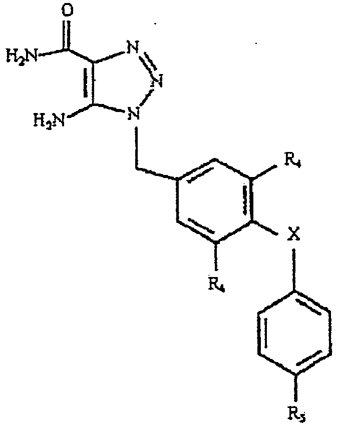 wherein X is CH₂, S, O or CO ; R₄ is Cl, CF₃, Br or CH₃ ; R₅ is Cl, Br or NO₂.
(4) In particular, the compound is 5-amino-1-[3, 5-dichloro-4-(4-chlorobenzoyl chloride) benzyl)-1H-1, 2, 3-triazole-4-carboxamide, i.e. carboxyamidotriazole (CAI) ;
(5)Also included are the salts of these compounds mentioned above, such as hydrochloride, sulfate, acetate and so on.

The instant invention provide use of above mentioned compounds as TNF-α and IL-l inhibitors and morphoine in preparation of a medicament for treating painful diseases caused by any reason and/or inflammatory diseases involving inflammatory cytokines, as well as other related inflammatory immune-mediated diseases, wherein said inflammation may be any acute or chronic inflammation caused by any reason; wherein said diseases include but not limited to rheumatic disease, rheumatoid arthritis, Adult Onset Still's Disease, Sjogren syndrome, systemic lupus erythematosus and related syndrome, arthritis of vertebral column, rigidity arthritis, psoriatic arthritis, bowel arthritis, reactive arthritis, and other undefined arthritis, scleroderma, mixed connective tissue disease and overlap syndrome, undifferentiated connective tissue disease, polymyositis and dermatomyositis, vasculitic syndrome, giant cell arteritis and polymyalgia rheumatica, Wegener granulomatosis, Churg-Strauss vasculitis, polyarteritis nodosa and related syndrome, microscopic polyangiitis, ANCA related polyangitis, Takayasu arteritis, Behcet disease, vasculitis of the skin, crystal arthrosis(including arthrolithiasis, calcium diphosphate precipitation, basic calcium phosphate crystal precipitation and other crystal arthrosis), amyloidosis, autoimmune liver disease, sarcoidosis, Fibromyalgia and Chronic fatigue syndrome, multicentric reticulo-histiocytosis, panniculitis, Lyme disease, Ormond's syndrome, osteoarthritis, diffusibility idio- hyperosteosis, osteitis deformans, ASPHO syndrome, relapsing polychondritis, osteonecrosis, heritage constitutive protein disease, Kashin-Bek syndrome and so on, Arthritis or secondary bone arthritis ; Said diseases include tumor, rheumatic disease, rheumatoid disease or collagen disease, as well as Septic shock, adult distress of respiratory (ARDS), acquired immunodeficiency (AIDS), injury of blood vessel/ atherosclerosis, type I diabetes, Kawasaki disease, leprosy, disseminated sclerosis, chronic anemia, ultraviolet radiation, HP gastritis / canker, Brazilian blastomycosis, septic melioidosis, cardiac failure, familial Mediterranean fever, toxic shock, chronic fatigue, homograft rejection, Graft-versus-host disease, schistosomiasis and so on.

In particular, the pharmaceutical composition compound could be in the form of a kit which includes instruction.

The pharmaceutical composition or kit include therapeutically effective amount of CAI or pharmaceutically acceptable salts, analogs or derivatives thereof, as well as therapeutically effective amount of morphine.

### Brief Description of the Figures

Fig.1 : Analgesic effect of CAI and morphine determined in formalin test
Fig.2 : Analgesic effect of CAI and morphine determined in hot plate test Fig.3 : Analgesic effect of CAI and morphine determined in radiant heat stimulus method
Fig.4 : Analgesic effect of CAI determined in mouse writhing test
Fig.5 : Anti-inflammatory effect of CAI determined in croton oil-induced ear edema mouse model.
Fig.6 : Effect of CAI on amount of TNF-α in arthritis rats' claws
Fig.7 : Effect of CAI on amount of IL-1β in arthritis rats' claws
Fig.8 : Effect of CAI on amount of TNF-α in arthritis rats' blood serum
Fig.9 : Effect of CAI on amount of IL-1β in arthritis rats' blood serum

### Embodiments

The invention will be further illustrated by the following examples, which are only intended to illustrate the present invention without limiting its scope in any way.

### Example 1 The effect of CAI on drug addiction caused by morphine

### Methods:

160 Kunming mice (18-22g, half male and half female) were divided into 4 groups randomly, with 40 mice per group:
1. Control group with solvent, PEG400/N.S. (10µl/g/day PEG400, (ig), 4 days);
2. Group treated with CAI, CAI/N.S. (CAI is resolved in PEG400 to reach a final concentration of 2mg/kg, 20mg CAI/kg/day was administered (ig) for 4 days, N.S. was taken according to morphine administration in table 1);
3. Group treated with morphine, PEG400/morphine (10µl/g/day PEG400 was administered (ig) for 4 days, morphine was administered according to the table
   below);
4. Group treated with morphine and CAI, CAI/morphine (CAI was administered according to dosage regimen of the group treated with CAI, and morphine was administered according to dosage regimen of the group treated with morphine).

**Table 1 Dosage regimen of morphine in the experiment for investigating the effect of CAI on drug addiction caused by morphine**

| time | 9 : 00 | 21 : 00 |
|---|---|---|
| Day 1 | 25 mg/kg | 25 mg/kg |
| Day 2 | 50 mg/kg | 50 mg/kg |
| Day 3 | 100 mg/kg | 100 mg/kg |
| Day 4 | 150 mg/kg | 150 mg/kg |
| Day 5 | 75 mg/kg | |

4 hours after the morphine administration on the fifth day, animals of each group was injected (ip) with 1mg/kg naloxone and then put into a tank (5000ml) immediately. The jump frequency in 30 minutes was recorded. Two-factor analysis of variance by SPSS was performed to analyse the experiment results.

### Results:

**Table 2 The effect of CAI on drug addiction caused by morphine**

| Groups | Mean number of jump |
|---|---|
| CAI/morphine | 43±41 |
| PEG400/morphine | 41±58 |
| CAI/N.S. | 5±14 |
| PEG400/N.S.control | 1±5 |

Two-factor analysis of variance by SPSS indicates that, significant difference between the groups treated with morphine (PEG400/morphine group and CAI/morphine group) and the control group (PEG400/N.S.) was observed (p<0.001). No significant difference was observed between the group treated with CAI alone and the control group (p=0.802). There is no significant difference between the CAI/morphine group and the PEG400/morphine group either, which indicates that treatment with morphine causes obvious withdrawal syndrome, which is not affected by CAI. This test demonstrates that long-term use of CAI does not cause addiction and the withdrawal syndrome in long term treated animals can not be induced by naloxone. It is also suggested that, mechanisms of the analgesic effect may be different between CAI and morphine.

### Example 2 The analgesic effect of CAI and morphine determined in the formalin test

### Methods:

320 Kunming mice (18-22g, half male and half female) were divided into 4 groups randomly with 80 animals in each group.
1. Control group with dissolvent (PEG400/N.S.): 10µl PEG400 was administered (ig) twice with the interval of 24 hours. The pain intensity test was carried out 8 hours after the second administration. 200µl N.S. is injected (sc) 5 minutes before the test.
2. Morphine/PEG400 group: 10µl PEG400 was administered (ig) twice with the interval of 24 hours. The pain intensity test was carried out 8 hours after the second administration. Morphine (2mg/kg body weight) was injected (sc) 5 minutes before the test.
3. CAI/N.S. group: CAI (20mg/kg body weight, 2mg/ml) was administered (ig) twice with the interval of 24 hours. The pain intensity test was carried out 8 hours after the second administration. 200µl N.S. was injected (sc) 5 minutes before the test.
4. CAI/morphine group: CAI (20mg/kg body weight, 2mg/ml) was administered (ig) twice with the interval of 24 hours. The pain intensity test was carried out 8 hours after the second administration. Morphine (2mg/kg body weight) was injected (sc) 5 minutes before the test.

5 minutes after the morphine or N.S. injection, 20µl 2% formalin was injected (sc) into the subplantar space of the hind paw. The animals were placed immediately in a glass cylinder hanging on a holder, with a mirror placed below with 30° slope to observe the paw responses. The pain responses of the animals with injected paws were recorded; a pain ranking was recorded as follows: 3 = the injected paw is licked, bitten, or shaken; 2 = the injected paw is elevated and is not in contact with any other surface; 1 = the injected paw has little or no weight on it; 0 = the injected paw is not favored.

The duration of each of the 4 behavioral categories was recorded. The observation lasted for 15 minutes. A pain intensity score was calculated by multiplying the amount of time spent in each category by its assigned category score and adding these products together.

### Results:

**Table 3 The analgesic effect of CAI and morphine determined in the formalin test**

| Groups | Pain intensity score(mean±std) |
|---|---|
| CAI/morphine | 318 ± 308 |
| PEG400/morphine | 756 ± 441 |
| CAI/N.S. | 1007 ± 255 |
| PEG400/N.S. | 1133 ± 185 |

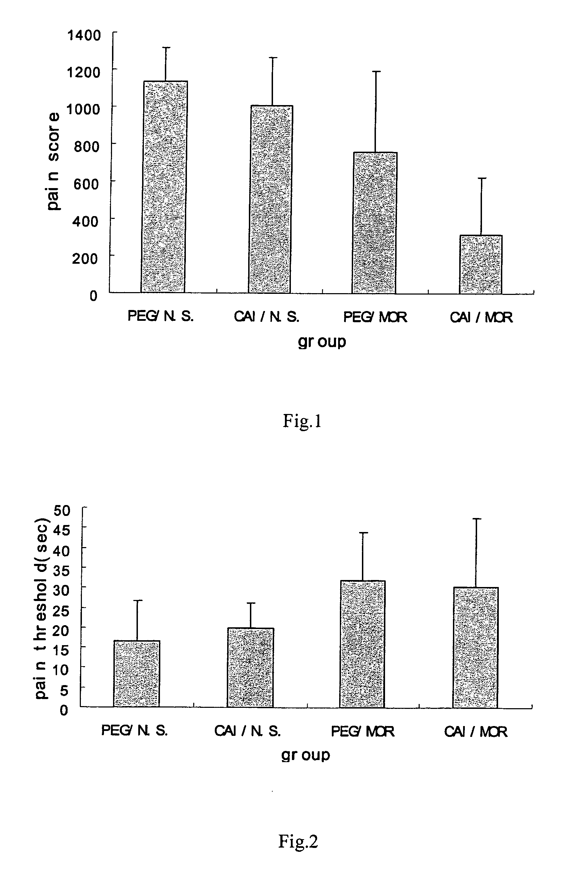

Figure 1 shows the analgesic effect of CAI and morphine determined in the formalin test; MOR stands for morphine. Two-factor analysis of variance by SPSS was performed. Compared with the control group, the pain scores of groups treated with CAI (p<0.001) and morphine (p<0.001) decrease significantly, indicating both of the drugs have potent analgesic effects. The score of the combination group is even lower, indicating the significant synergistic action of morphine and CAI ((P=0.004)).

### Example 3 The analgesic effect of CAI and morphine determined in hot plate test

### Methods:

Kunming mice (18-22g, female) were randomly divided into 4 groups with 20 animals per group.
1. Control group with dissolvent: 10µl PEG400 was administered (ig) twice with the interval of 24 hours. The pain intensity test was carried out 8 hours after the second administration. 200µl N.S. was injected (sc) 5 minutes before the test.
2. Morphine/PEG400 group: 10µl PEG400 was administered (ig) twice with the interval of 24 hours. The pain intensity test was carried out 8 hours after the second administration. Morphine (2mg/kg body weight) was injected (sc) 5 minutes before the test.
3. CAI/N.S. group: CAI (20mg/kg body weight, 2mg/ml) was administered (ig) twice with the interval of 24 hours. The pain intensity test was carried out 8 hours after the second administration. 200µl N.S. was injected (sc) 5 minutes before the test.
4. CAI/morphine group: CAI (20mg/kg body weight, 2mg/ml) was administered (ig) twice with the interval of 24 hours. The pain intensity test was carried out 8 hours after the second administration. Morphine (2mg/kg body weight) was injected (sc) 5 minutes before the test.

The mice were placed onto a hot plate (53±0.5□) and the latency periods (second) of hopping responses were recorded as the pain threshold index.

### Results:

**Table 4 The analgesic effect of CAI and morphine determined in hot plate test**

| **Groups** | **Pain threshold**(sec, mean±std) |
|---|---|
| CAI/morphine | 30.3±17.3 |
| PEG400/morphine | 31.9±12.1 |
| CAI/N.S. | 19.9±6.2 |
| PEG400/N.S. | 16.7±10.2 |

Figure 2 shows the analgesic effect of CAI and morphine determined in the hot plate test. Two-factor analysis of variance by SPSS was performed. Morphine (p=0.002) but not CAI (p=0.834) extends the pain response time very significantly, and the combination effect is not obvious, indicating that CAI neither have obvious analgesic effect alone nor affect the analgesic effect of morphine in this animal model.

### Example 4 The analgesic effect of CAI and morphine determined in radiant heat test

### Methods:

The radiant heat dolorimeter (Collumbus Instruments) is used in this model with the tail-flick mode and the exposure rate of 20. The latency periods (TFL) starting from the beginning of exposure to the tail-flick responses were recorded as the pain threshold. The exposure time lasted for 16 seconds.

Kunming mice (18-22g, male) are tested for the basic pain threshold before the test with drug administration, and those with pain threshold longer than 16 seconds are excluded. The selected mice are divided into 4 groups with 15 animals per group.
1. Control group with dissolvent: 10µl PEG400 was administered (ig) twice with the interval of 24 hours. The pain intensity test was carried out 8 hours after the second administration. 200µl N.S. was injected (sc) 5 minutes before the test.
2. Morphine/PEG400 group: 10µl PEG400 was administered (ig) twice with the interval of 24 hours. The pain intensity test was carried out 8 hours after the second administration. Morphine (2mg/kg body weight) was injected (sc) 5 minutes before the test.
3. CAI/N.S. group: CAI (20mg/kg body weight, 2mg/ml) was administered (ig) twice with the interval of 24 hours. The pain intensity test was carried out 8 hours after the second administration. 200µl N.S. was injected (sc) 5 minutes before the test.
4. CAI/morphine group: CAI (20mg/kg body weight, 2mg/ml) was administered (ig) twice with the interval of 24 hours. The pain intensity test was carried out 8 hours after the second administration morphine (2mg/kg body weight) was injected (sc) 5 minutes before the test.

The pain threshold of each mouse was tested before and after morphine or N.S. injection. The values after drug adminstration minus those before drug administration were considered to be the changing index of pain threshold.

### Results:

**Table 5 The analgesic effect of CAI and morphine determined in radiant heat test**

| Groups | Pain threshold change(seconds, mean±std) |
|---|---|
| CAI/morphine | 7.66±4.63 |
| PEG400/morphine | 6.95±5.69 |
| CAI/N.S. | 2.56±2.62 |
| PEG400/N.S. | 2.36±1.61 |

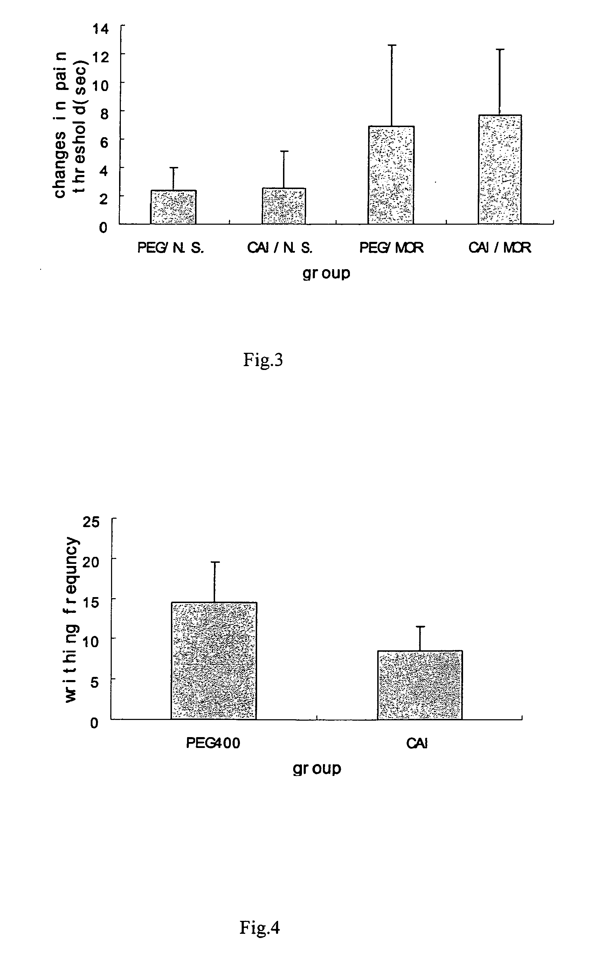

Figure 3 shows the analgesic effect of CAI and morphine determined in the radiant heat test. Two-factor analysis of variance by SPSS was performed. Compared with the control group, morphine (p<0.001) but not CAI (p=0.690) extended the pain threshold time very significantly, while the combination effect was not obvious (P=0.820), indicating that CAI neither have obvious analgesic effect alone nor affect the analgesic effect of morphinein this animal model.

### Example 5 Analgesic activity of CAI and its analogues determined in mouse writhing tests

### Methods:

Kunming mice (18-22g, male) were randomly divided into several groups with 20 animals in each group.
1. Control group: PEG400 was administered (ig) for 4 days, 0.1ml/10g body weight.
2. Test groups: CAI and its analogues (compounds with structural formula of A, R₁=Cl or H, R₂= Cl, H, Br, CH₃ or CF₃, R₃=Cl, Br, CF₃, NO₂ or CN, X=O, S or C=O;The compounds are listed in Table 6 (A1~A10)) were administered (ig) for 4 days (20mg/kg body weight, 2mg/ml).

The pain intensity test was carried out 8 hours after the last drug administration. 0.6% acetic acid (0.1ml/10g body weight) was injected (ip). The animals were put into a glass cylinder immediately. The writhing frequency in the following 5-15 minutes was recorded.

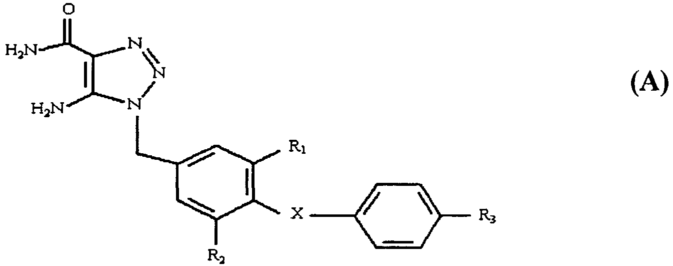

### Results:

**Table 6 Analgesic activity of CAI and its analogues determined in mouse writhing tests**

| **Groups** | | | | | **Writhing frequency** mean±std |
|---|---|---|---|---|---|
| PEG400 | | | | | 14.6±4.9 |
| CAI | | | | | 8.1±2.3 |
| CAI analogs | R1 | R2 | X | R3 | |
| Al | Cl | Cl | C=O | Cl | 8.6±3.0 |
| A2 | Cl | H | S | Cl | 7.8±1.4 |
| A3 | H | CF₃ | S | Cl | 7.9±2.2 |
| A4 | Cl | CH₃ | C=O | CF₃ | 8.9±2.5 |
| A5 | Cl | Cl | C=O | CN | 8.6±2.0 |
| A6 | Cl | Br | C=O | Cl | 7.9±3.0 |
| A7 | Cl | Cl | O | Br | 7.2±2.1 |
| A8 | Cl | Cl | C=O | Br | 8.8±2.0 |
| A9 | Cl | Cl | O | NO₂ | 7.6±1.6 |
| A10 | Cl | CH₃ | O | Cl | 7.2±1.6 |

Figure 4 show the analgesic activity of CAI and its analogues determined in mouse writhing tests. Independent-sample t test by SPSS was performed. It is shown that CAI (p<0.001) has obvious analgesic effect in this animal model. According to the data in Table 6, analogues of CAI (compounds A1~A10) also have obvious analgesic effect in this animal model.

### Example 6 Effect of CAI and its analogs determined in croton oil-induced ear edema mouse model

### Method:

Male Kunming mice, weighted 18-22g, were randomly divided to the following experiment groups, 20 mice/group:
1. Negative control: normal saline was injected (ig), 0.1ml/10g/day for 3 consecutive days.
2. Vehicle control: PEG400 was injected (ig), 0.1mg/10g/day for 3 consecutive days.
3. Positive control: Dexamethasone (0.2mg/ml) was injected (ip), 0.1ml/10g/day for 3 consecutive days.
4. Test group: CAI or its analogs (2 mg/ml, analogs are as described in example 5) was administered (ig), 0.1ml/10g /day for 3 consecutive days.

8 hours after the last administration, 20 µl of croton oil solution (2%) was applied to the right ear of each mouse. The left ear remained untreated as control. The animals were sacrificed 4 hours later, and a piece of tissue was punched from the same position of the treated and untreated ear. The tissues were weighed on a 0.1 mg balance. The difference in weight between the two pieces of tissue of each mouse was taken as a measure of swelling intensity.

### Results:

**Table 7 Effect of CAI and its analogs determined in croton oil-induced ear edema mouse model**

| **Group** | | | | | **Swelling intensity** **(mg)** **mean±std** |
|---|---|---|---|---|---|
| N.S. | | | | | 12.5 ± 0.6 |
| PEG400 | | | | | 11.6 ± 0.4 |
| dexamethasone | | | | | 1.5 ± 0.4 |
| CAI | | | | | 7.8 ± 0.2 |
| CAI analogs | R1 | R2 | X | R3 | |
| Al | Cl | Cl | C=O | Cl | 8.0 ± 0.8 |
| A2 | Cl | H | S | Cl | 7.8 ± 0.4 |
| A3 | H | CF₃ | S | Cl | 7.9 ± 0.2 |
| A4 | Cl | CH₃ | C=O | CF₃ | 8.0 ± 0.5 |
| A5 | Cl | Cl | C=O | CN | 8.6 ± 0.6 |
| A6 | Cl | Br | C=O | Cl | 7.9 ± 0.3 |
| A7 | Cl | Cl | O | Br | 8.2 ± 0.6 |
| A8 | Cl | Cl | C=O | Br | 8.8 ± 0.2 |
| A9 | Cl | Cl | O | NO₂ | 8.6 ± 0.6 |
| A10 | Cl | CH₃ | O | Cl | 8.2 ± 0.6 |

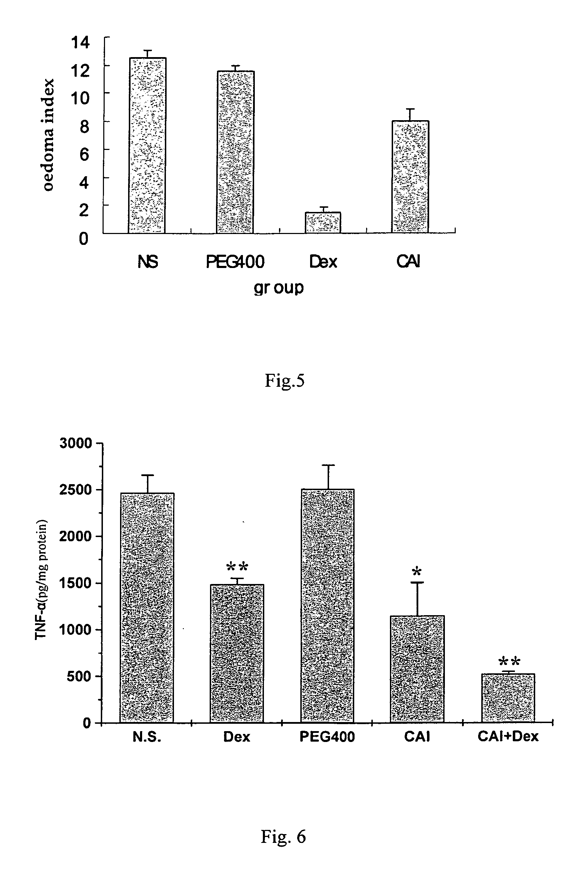

Figure 5 shows the effect of CAI and its analogs in croton oil-induced ear edema mouse model. Independent-sample t test by SPSS was performed. It is shown that CAI has obvious anti-inflammatory effects (P=0.028). As indicated by data of table 7, the analogs of CAI (compound A1-A10) have similar activities as that of CAI in this model.

### Example 7 Effect of CAI on granulomatous tissue formation

### Method:

44 rats (160g) were randomly divided into 4 groups (11 rats/group). Animals were slightly anaesthetized by ether. After sterilization, the groin was cut bilaterally. A sterile tampon (50mg) was implanted (sc). On the same day, the following treatments were administered: N.S. (i.p., control group), 0.067 mg/kg/day dexamethasone (i.p., positive control), 20mg/kg/day CAI (p.o., dissolved in PEG 400, test group) and 0.067 mg/kg/day dexamethasone + 20mg/kg/day CAI (p.o., dissolved in PEG 400, combination group). After 7 days administration, the animals were sacrificed. The cotton was removed. The granulomatous tissue was dried at 60°C oven for 12 hr and weighed. Increment in the dry weight of the tissue was taken as measure of granuloma formation. The Increment was adjusted for respective body weight, and the mean value for each group was calculated.

### Results:

**Table 8 Effect of CAI on granulomatous tissue formation**

| Group | granulomatous tissue:bady weight ratio(mean±std) |
|---|---|
| Control | 0.075 ± 0.00667 |
| Dexamethasone | 0.064 ± 0.00495 |
| CAI | 0.060 ± 0.00479 |
| CAI + Dexamethasone | 0.054 ± 0.00405 |

Statistical analysis (two-way ANOVA) revealed that the effects of dexamethasone (p=0.049) and CAI (p=0.037) on granulomatous tissue formation are both significant, and a synergy in anti-inflammatory activity between the two was observed. It is suggested that CAI is potent in inhibiting granulomatous tissue formation induced by chronic inflammation. The potency of CAI is comparable to that of dexamethasone, or even more potent than dexamethasone, and can enhance the effect of dexamethasone.

### Example 8 Effect of CAI on adjuvant induced arthritis (AA) of rat

### Method:

84 male Wistar rats (160±10g) were used. A control group of 14 rats was randomly picked and N.S. (0.1ml/200g) was injected into the plantar region of the left hind paw. Adjuvant arthritis was induced in other animals by injection of 0.1 ml of Freund's complete adjuvant (FCA) into the plantar region of the left hind paw. After model establishment, the 70 rats were randomly divided into 5 groups (14/group):
1. Negative control (AA + NS): N.S., ig, 0.1ml/100g.
2. positive control (AA + Dex): dexamethasone, i.p., 0.067 mg/kg/day.
3. Vehicle control (AA + PEG400): PEG400, ig, 0.1ml/100g.
4. CAI (AA + CAI): 20mg/ml CAI, ig, 0.1ml/100g/day (20mg/kg/day).
5. Combination (AA + CAI + Dex): dexamethasone, 0.067 mg/kg/day (i.p.) plus C AI 20 mg/kg/day (p.o.).

Normal volumes of left and right hind paw were measured before adjuvant injection. Volume of left hind paw was measured on day 2, 5, 8, 11, 14, 17, 20, 23, 26, 29 after adjuvant injection; Right hind paw volume was measured on day 14, 17, 20, 23, 26, 29. The result is shown below.

### Result:

**Table 9 Effect of CAI on primary paw oedema**

| Group | N | Paw oedema(%), mean±std | | | |
|---|---|---|---|---|---|
| | | 1d | 2d | 5d | 8d |
| AA+NS | 14 | 129.9±20.0 | 132.4±27.1 | 147.6±15.4 | 143.6±19.0 |
| AA+Dex 11 | | 111.3±13.6* | 121.9±16.0* | 107.4±12.5** | 103.6±14.0** |
| AA + | 14 | 126.6±12.5 | 146.9±15.0 | 133.4±24.38 | 138.6±15.8 |
| PEG400 | | | | | |
| AA + CAI | 12 | 116.2±17.6# | 131.9±23.0# | 122.4±12.4# | 121.6±21.6# |
| AA + CAI+Dex | 12 | 109.1±12.3# | 124.6±15.3# # | 105.4±16.8## | 103.6±11.6## |

| Group | N | Paw oedema(%), mean±std | | | |
|---|---|---|---|---|---|
| | | 11d | 14d | 17d | 20d |
| AA+NS | 14 | 124.3±12.3 | 112.3±14.0 | 112.9±21.8 | 108.4±15.7 |
| AA+Dex | 11 | 100.3±21.0* * | 100.0±11.3* | 95.3±15.7* | 83.9±24.0** |
| AA + PEG400 | 14 | 149.3±12.5 | 126.2±16.9 | 112.3±13.8 | 104.4±18.2 |
| AA+CAI | 12 | 129.3±23.5# # | 112.0±15.7# | 104.3±20.7# | 92.2±16.0# |
| AA + CAI+Dex | 12 | 99.2±17.6## | 99.9±23.0## | 92.4±11.4## | 81.6±23.0## |

| Group | N | Paw oedema(%), mean±std | | | |
|---|---|---|---|---|---|
| | | 23d | 26d | 29d | |
| AA + NS | 14 | 106.6±11.3 | 103.6±19.0 | 99.5±14.0 | |
| AA+Dex | 11 | 80.4±17.8** | 78.6±23.1** | 73.9±18.0** | |
| AA + PEG400 | 14 | 100.4±15.2 | 105.6±14.6 | 100.5±15.6 | |
| AA + CAI | 12 | 92.4±15.4# | 88.6±13.4## | 86.9+23.1## | |
| AA + CAI+Dex | 12 | 74.2±13.1## | 72.9±21.2## | 72.4±11.5## | |

| | | | | | |
|---|---|---|---|---|---|
| Note: compared with N.S. * P< 0.05, ** P< 0.01;: compared with PEG400, #P< 0.05, ##P< 0.01. | | | | | |

**Table 10 Effect of CAI on secondary paw oedema**

| Group | Paw oedema(%), mean±std | | | | | |
|---|---|---|---|---|---|---|
| | 14d | 17d | 20d | 23d | 26d | 29d |
| AA + NS | 3.3±2.0 | 4.9±1.1 | 12.8±5.7 | 17.6±1.3 | 16.6±1.6 | 10.5±4.7 |
| AA + Dex | 1.1±1.0* | 1.9±1.4* | 2.8±0.9* | 4.6±1.4** | 3.6±1.7** | 2.5±1.7* |
| AA + PEG400 | 4.3±1.1 | 6.3±1.5 | 15.8±3.7 | 17.8±3.4 | 17.6±2.9 | 12.4±2.8 |
| AA + CAI | 2.3±1.0# | 3.1±0.8 | 8.8±3.2# | 8.6±2.3# | 8.6±1.9# | 6.5±2.7# |
| AA + | 1.0±1.0* | 1.7±1.4* | 1.8±0.9* | 3.5±0.4** | 3.3±1.3** | 1.5±1.1* |
| CAI + Dex | | | | | | |

| | | | | | | |
|---|---|---|---|---|---|---|
| Note: compared with N.S. * P< 0.05, ** P< 0.01;: compared with PEG400, #P< 0.05. | | | | | | |

Statistical analysis (two-way ANOVA) revealed that the effects of CAI and dexamethasone are both significant when compared with control group, and an additive effect was evident when administered concomitantly. CAI inhibits primary and secondary paw oedema in rats, significantly alleviating rat arthritis.

### Example 9 Effect of CAI on cytokine levels in paw tissue and serum of adjuvant arthritis rat

### Method:

The adjuvant arthritis rat was prepared as described in example 8. Treatment was perfomed as described in example 8. Blood was collected from eye socket on day 29 and left in room temperature to allow coagulating. Then serum was separated by centrifugation at 3000rpm for 10 min and kept in -20□. After animals were sacrificed, the subcutaneous tissue of the right hind paw and that surrounding the tarsotibial joints was removed, broke into pieces, and placed in 5 ml normal saline (0.9%). The tissues were homogenized in a high speed homogenizer. Then the homogenate was centrifuged at 4°C and 10, 000rpm for 15 min. The supernatants were collected and stored at -20°C until further analysis.

IL-1β and TNF-α levels in paw tissue and serum were measured using specific rat ELISA kits according to the manufacturer's instructions.

### Result:

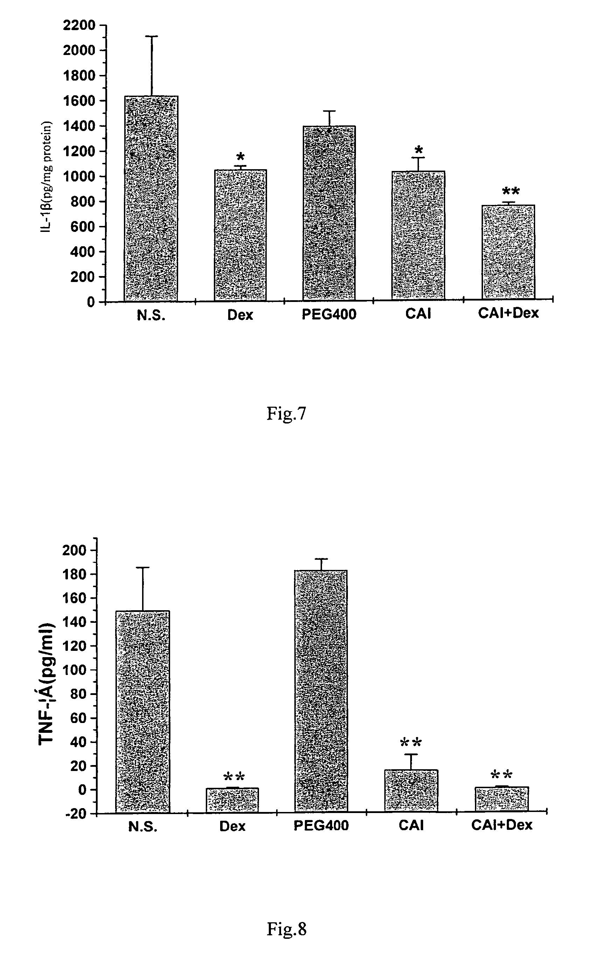

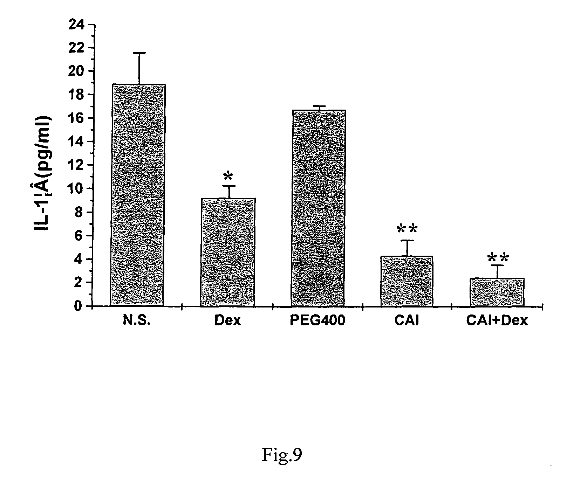

CAI significantly reduced TNF-α and IL-1β levels in paw tissue and serum. Statistical analysis (two-way ANOVA) revealed that the effects of CAI, dexamethasone and the combination of the two are significant when compared with control group, and a synesthetic effect was present when administered concomitantly, as illustrated in Fig 6, 7, 8, 9.

In another set of experiments, CAI was administered in combination of its analogs (CAI-imid, CAI hydrochlorate, CAI sulfate, CAI acetate) and the inhibitory effect of these combinations on serum levels of TNF-α and IL-1β are obvious and even more pronounced. Thus, it can be inferred that the CAI and its analogs can be used in combination to serve as TNF-α and IL-1β inhibitor.

To summarize, 2 types of animal models were employed in this invention to verify the analgesic effect of CAI and its analog: CNS pain models (hot plate model and radiant heat model) and inflammatory or injury related pain models (formalin test and acetic acid induced writhing). It can be seen from CAI is an active analgesic agent in peripheral pain models, but futile in CNS pain models. In addition, CAI enhanced the effect of morphine (a CNS analgesic agent) in peripheral pain model (formalin test). These results suggest CAI an active peripheral analgesic agent.

Moreover, the effects of CAI on acute and chronic phases of inflammation were investigated using 3 animal models: one is acute inflammation model (croton oil-induced ear edema), the other two are sub-acute and chronic inflammation model (granuloma formation and adjuvant-induced rat arthritis) in the present invention. Anti-inflammatory activity of CAI and analogs thereof was proven in all these models. It is noted that CAI significantly inhibits the primary and secondary paw oedema in rats, significantly alleviate rat arthritis. It has been well established that cytokines such as TNF-α and IL-1β are closely related to the onset of rheumatoid arthritis (RA). TNF-α can be found in Synovial cells of RA model, especially in the joint of blood vessel and cartilage. It plays a critical role in synovial membrane inflammatory reactions and induces joint destructions. IL-1β activates osteoclast and inhibits proteoglycan and collagen synthesi, leading to resorption of bone and cartilage. These cytokine are known as "twins cytokines" in RA and they are "axis of evil" in RA. The data above indicate that CAI can reduce TNF-α and IL-1β levels in both arthritic paws and in serums to a extent comparable to dexamethasone, indicating that CAI may act as inhibitor for TNF-α and IL-1β. With the evidence, it can be inferred thatCAI is a promising compound that can be used in treatment of rheumatic diseases that may involve muscle, joint and soft tissue and with a primary symptom of pain.

This invention reveals for the first time that CAI, which has long been recognized as an anti-cancer drug, is a potent inhibitor of pro-inflammatory cytokines (TNF-α and IL-1β) and that it has analgesic and anti-inflammatory effect. This invention is considered to be of particular importance, such as:
1. It is known in the art that, patients with advanced cancers are usually suffered from severe cancer pain. CAI is the first compound, as shown by this invention that acts as both cancer blocker and painkiller. This particular feature will promote its application in treatment of cancers.
2. This invention demonstrates the possibility of developing CAI and its analogs or derivates as a novel class of anti-inflammatory drugs. The indications of CAI and its analogs or derivates would not only be limited to cancers, but also other diseases and conditions that are related to inflammation or pain, includdingbut not limited to rheumatic disease, rheumatoid disease and other collagenosis etc..
3. The experiment results indicate that the analogs and derivates of CAI are also active anti-inflammatory and analgesic agents as CAI. These compounds form a pool for further screening of painkillers and anti-inflammation agents.
4. CAI is proved to be synergy with known analgesic agents (e.g, morphine) or anti-inflammatory agents (e.g, dexamethasone) when administered concomitantly.

One skilled in the art should also understand that, the present invention to treatment of pains and/or inflammations whatever the causes are. The inflammations herein refer to any acute or chronic inflammations induced by any cause, including but not limited to rheumatic disease, rheumatoid arthritis, adult still disease, Sjogren syndrome, systemic lupus erythematosus and related syndrome, vertebral column arthritis, rigidity arthritis, psoriatic arthritis, bowel arthritis, reactive arthritis and other arthritis, scleroderma, mixed connective tissue disease and overlap syndrome, undifferentiated connective tissue disease, polymyositis and dermatomyositis, vasculitic syndrome, giant cell arteritis and polymyalgia rheumatica, Wegener granulomatosis, Churg-Strauss vasculitis, polyarteritis nodosa and related syndrome, microscopic polyangiitis, ANCA related polyangitis, Takayasu arteritis, Behcet disease, Vasculitis of the skin, crystal arthrosis(include arthrolithiasis, calcium diphosphate precipitation, basic calcium phosphate crystal precipitation and other crystal arthrosis), amyloidosis, autoimmune liver disease, sarcoidosis, Fibromyalgia and Chronic fatigue syndrome, multicentric reticulo-histiocytosis, panniculitis, Lyme disease, Ormond's syndrome, osteoarthritis, diffusibility idio- hyperosteosis, osteitis deformans, ASPHO syndrome, relapsing polychondritis, osteonecrosis, heritage constitutive protein disease, Kashin-Bek syndrome and secondary arthritis. Also include tumor, rheumatic disease, rheumatoid disease or collagen disease and septic shock, adult distress of respiratory(ARDS), acquired immunodeficiency(AIDS), injury of blood vessel/atherosclerosis, type I diabetes, Kawasaki disease, leprosy, disseminated sclerosis, chronic anemia, ultraviolet radiation, HP gastritis / canker, Brazilian blastomycosis, septic melioidosis, cardia failure, familial Mediterranean fever, toxic shock, chronic fatigue, homograft rejection, Graft-versus-host disease, schistosomiasis.

### Applicability

The present invention has demonstrated the inhibitory activity of CAI and its analogs/derivates towards inflammatory cytokines TNF-α and IL-1 and this particular feature makes CAI and its analogs/derivates a promising compound class that can be developed as a treatment option of mammalian pains of any kind and/or cytokine-involved inflammations .

## Claims

1. A pharmaceutical composition for its use in the treatment of TNF-α and/or IL-1β mediated painful and/or inflammatory diseases, comprising an effective amount of a compound of formula: 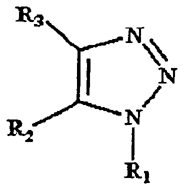 where, R₁ is 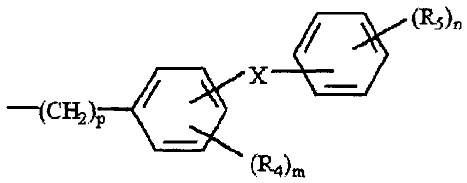 wherein, p is an integer from 0 to 2 ; m is an integer from 0 to 4 ; n is an integer from 0 to 5 ; X represents O, S, SO, SO₂, CO, CHCN, CH₂ or C=NR₆, wherein R₆ may be H, (C₁-C₆) alkyl, hydroxy, (C₁-C₆) alkoxyl, amino, (C₁-C₆) alkylamino, dialkylamino or cyano ;
R₄ and R₅ are independently halogen, cyano, trifluoromethyl, (C₁-C₆) alkyl acyl, nitro, (C₁-C₆)alkyl, (C₁-C₆) alkoxyl, carbonyl, alkyl ester, trifluoromethoxyl, acetyl amino, (C₁-C₆) alkyl thiol, (C₁-C₆) alkyl sulfuryl, trichloroethylene, trifluoromethylthio, trifluoromethylsulfinyl, and trifluoromethylsulfuryl;
R₂ is amido, (C₁-C₆)alkylamino, dialkylamino, acetyl amino, acetylimino, acyl ureido, formamido, formylimino or guanidino;
R3 is amino formyl, cyano, formamyl, imino or N-hydroxy formamyl;
or a pharmaceutically acceptable salt thereof;
and an effective amount of morphine.

2. The pharmaceutical compositon according to claim 1, wherein n and m are independently 0, 1 or 2; p is 1; X is O, S, CO or CH₂;
R₄ is F, Cl, Br, methyl, trifluoromethyl, cyano methoxycarbonyl, trifluoromethoxy, trifluoromethylthio, nitro or trichloroethylene;
R₅ is F, Cl, Br, methyl, trifluoromethyl, cyano, alkyl ester, trifluoroethylene or nitro.

3. The pharmaceutical composition according to claim 1, having the structure 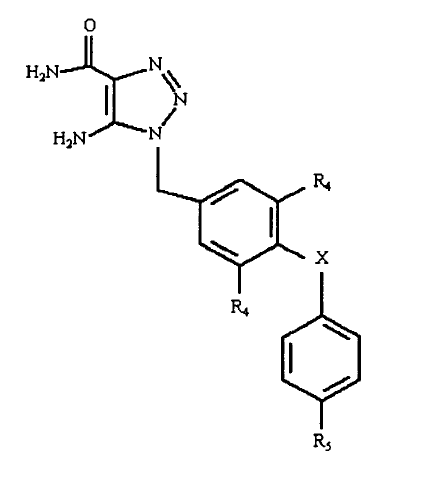 wherein X is CH₂, S, O or CO; R₄ is Cl, CF₃, Br or CH₃; R₅ is Cl, Br or NO₂.

4. The pharmaceutical composition according to claim 1, which are 5-amino-1-[3, 5-dichlorine-4(4-chlorobenzoyl chloride)) benzyl]-1H-1,2,3-triazole-4-benzamide, i.e, carboxyamidotriazole and its pharmaceutically acceptable salts.

5. The pharmaceutical composition according to any one of claims 1 to 4, wherein diseases are pains-related diseases, preferably selected from the group consisting of pains caused by inflammation, chemical or physical stimulus or hurt as well as bacterial infection.

## Patentansprüche

1. Pharmazeutische Zusammensetzung zur Verwendung bei der Behandlung von TNF-α- und/oder IL-1β-vermittelten schmerzhaften und/oder entzündlichen Erkrankungen, umfassend eine wirksame Menge einer Verbindung der Formel: 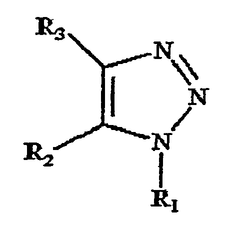 worin R₁ 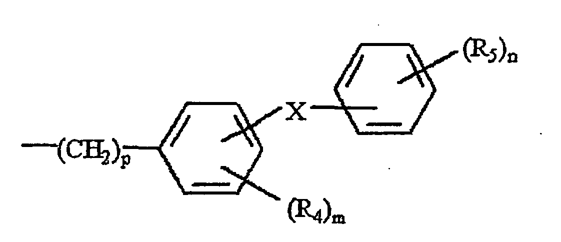 ist, wobei p eine ganze Zahl von 0 bis 2 ist, m eine ganze Zahl von 0 bis 4 ist, n eine ganze Zahl von 0 bis 5 ist, X O, S, SO₂, CO, CHCN, CH₂ oder C=NR₆ darstellt, wobei R₆ H, (C₁-C₆)-Alkyl, Hydroxy, (C₁-C₆)-Alkoxyl, Amino, (C₁-C₆)-Alkylamino, Dialkylamino oder Cyano sein kann,
R₄ und R₅ unabhängig voneinander Halogen, Cyano, Trifluormethyl, (C₁-C₆)-Alkylacyl, Nitro, (C₁-C₆)-Alkyl, (C₁-C₆)-Alkoxyl, Carbonyl, Alkylester, Trifluormethoxyl, Acetylamino, (C₁-C₆)-Alkylthiol, (C₁-C₆)-Alkyisulfuryl, Trichlorethylen, Trifluormethylthio, Trifluormethylsulfinyl und Trifluormethylsulfuryl sind,
R₂ Amido, (C₁-C₆)-Alkylamino, Dialkylamino, Acetylamino, Acetylimino, Acylureido, Formamido, Formylimino oder Guanidino ist,
R₃ Aminoformyl, Cyano, Formamyl, Imino oder N-hydroxyformamyl ist,
oder ein pharmazeutisch akzeptables Salz davon und eine wirksame Menge eines Morphins.

2. Pharmazeutische Zusammensetzung gemäß Anspruch 1, wobei n und m unabhängig voneinander 0, 1 oder 2 sind, p 1 ist, X O, S, CO oder CH₂ ist,
R₄ F, Cl, Br, Methyl, Trifluormethyl, Cyano, Methoxycarbonyl, Trifluormethoxy, Trifluormethylthio, Nitro oder Trichlorethylen ist,
R₅ F, Cl, Br, Methyl, Trifluormethyl, Cyano, Alkylester, Trifluorethylen oder Nitro ist.

3. Pharmazeutische Zusammensetzung gemäß Anspruch 1, welche die Struktur 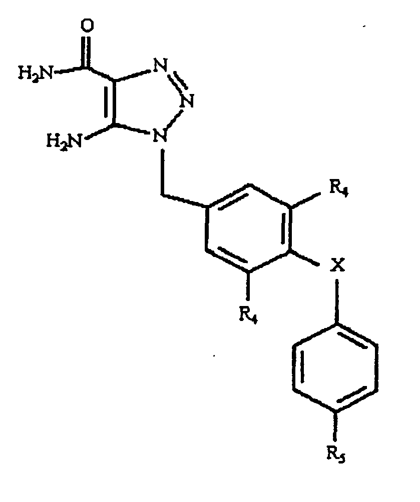 aufweist, wobei X CH₂, S, O oder CO ist, R₄ Cl, CF₃, Br oder CH₃ ist, R₅ Cl, Br oder NO₂ ist.

4. Pharmazeutische Zusammensetzung gemäß Anspruch 1, wobei es sich um 5-Amino-1-[3,5-dichlor-4(4-chlorbenzoylchlorid)benzyl]-1H-1,2,3-triazol-4-benzamid, d.h. Carboxyamidotriazol, und dessen pharmazeutisch akzeptable Salze handelt.

5. Pharmazeutische Zusammensetzung gemäß irgendeinem der Ansprüche 1 bis 4, wobei die Erkrankungen Schmerzerkrankungen sind, die bevorzugt aus der Gruppe bestehend aus durch Entzündung, chemischen oder physikalischen Stimulus oder Verletzung sowie bakterielle Infektion verursachten Schmerzen ausgewählt sind.

## Revendications

1. Composition pharmaceutique pour son utilisation dans le traitement de maladies douloureuses et/ou inflammatoires médiées par le TNF-α et/ou l'IL-1β, comprenant une quantité efficace d'un composé de formule : 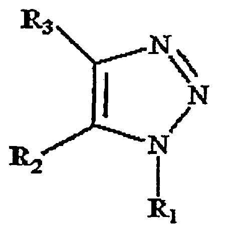 où, R₁ représente 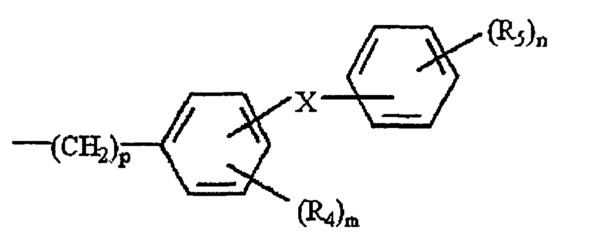 où, p représente un nombre entier de 0 à 2 ; m représente un nombre entier de 0 à 4 ; n représente un nombre entier de 0 à 5 ; X représente O, S, SO, SO₂, CO, CHCN, CH₂ ou C=NR₆,
où R₆ peut représenter H, un groupe (C₁-C₆)alkyle, hydroxy, (C₁-C₆)alcoxyle, amino, (C₁-C₆)alkylamino, dialkylamino ou cyano ;
R₄ et R₅ représentent indépendamment un atome d'halogène, un groupe cyano, trifluorométhyle, (C₁-C₆)alkylacyle, nitro, (C₁-C₆)alkyle, (C₁-C₆) - alcoxyle, carbonyle, ester d'alkyle, trifluorométhoxyle, acétylamino, (C₁-C₆)alkylthiol, (C₁-C₆)alkylsulfuryle, trichloroéthylène, trifluorométhylthio, trifluorométhylsulfinyle et trifluorométhylsulfuryle ;
R₂ représente un groupe amido, (C₁-C₆)alkylamino, dialkylamino, acétylamino, acétylimino, acyluréido, formamido, formylimino ou guanidino ;
R₃ représente un groupe amino-formyle, cyano, formamyle, imino ou N-hydroxy-formamyle ;
ou un sel pharmaceutiquement acceptable de celui-ci ;
et une quantité efficace de morphine.

2. Composition pharmaceutique selon la revendication 1, où n et m valent indépendamment 0, 1 ou 2 ; p vaut 1 ; X représente O, S, CO ou CH₂ ;
R₄ représente F, Cl, Br, un groupe méthyle, trifluorométhyle, cyano, méthoxycarbonyle, trifluorométhoxy, trifluorométhylthio, nitro ou trichloroéthylène ;
R₅ représente F, Cl, Br, un groupe méthyle, trifluorométhyle, cyano, ester d'alkyle, trifluoro-éthylène ou nitro.

3. Composition pharmaceutique selon la revendication 1, ayant la structure 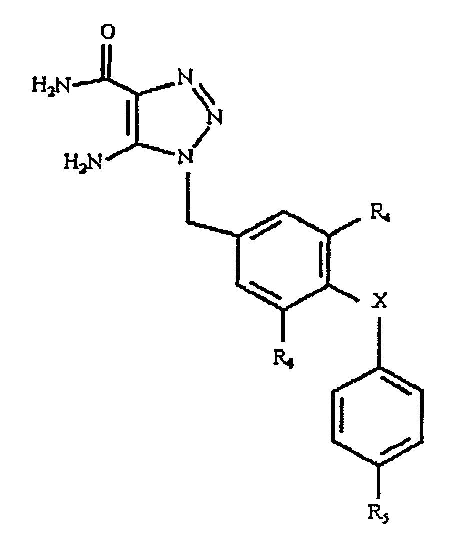 où X représente CH₂, S, O ou CO ; R₄ représente Cl, CF₃, Br ou CH₃ ; R₅ représente Cl, Br ou NO₂.

4. Composition pharmaceutique selon la revendication 1, qui est constituée de 5-amino-1-[3,5-dichlore-4-(chlorure de 4-chlorobenzoyle)benzyl]-1H-1,2,3-triazole-4-benzamide, c'est-à-dire carboxyamidotriazole et de ses sels pharmaceutiquement acceptables.

5. Composition pharmaceutique selon l'une quelconque des revendications 1 à 4, où les maladies sont des maladies associées à des douleurs, de préférence choisies dans le groupe constitué de douleurs provoquées par une inflammation, un stimulus chimique ou physique ou une blessure ainsi qu'une infection bactérienne.
